# EUROPÄISCHE PATENTANMELDUNG

(11) **EP 4 684 736 A1**
(43) Veröffentlichungstag der Anmeldung: **28.01.2026**
(21) Anmeldenummer: 25189867.2
(22) Anmeldetag: 16.07.2025
(51) Int. Cl.: A61B 8/08, A61B 8/12, A61B 8/00, A61B 17/34

(54) **VERFAHREN UND SYSTEM ZUM ERMITTELN EINER POSITION EINER KANÜLENSPITZE**

(30) Priorität: 23.07.2024 DE 102024120903
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Die Erfindung betrifft ein Verfahren zum Ermitteln einer Position einer Kanülenspitze im Inneren eines Körpers, aufweisend die Schritte: Einkoppeln eines Ultraschallsignals in den Körper, wobei das Ultraschallsignal mit einem auf den Körper aufliegenden Ultraschallkopf und entlang einer Bildebene eines bildgebenden Ultraschallverfahrens in den Körper eingekoppelt wird, wobei die Bildebene entlang einer Tiefenrichtung und einer Querrichtung erstreckt ist; Empfangen des Ultraschallsignals, wobei das Ultraschallsignal mit mehreren Sensoren empfangen wird, die an einer in dem Körper befindlichen Kanüle angebracht und in bekannten axialen Abständen zu der Kanülenspitze und zueinander entlang einer Längsachse der Kanüle angeordnet sind; Vergleichen der empfangenen Ultraschallsignale, wobei die empfangenen Ultraschallsignale im Hinblick auf ihre Signalstärken und ihre Signalverläufe mittels einer Auswerteeinrichtung verglichen werden, die mit den Sensoren verbunden ist; Ermitteln der Position der Katheterspitze in Bezug auf den Ultraschallkopf, wobei die Position in Abhängigkeit des Vergleichs der Signalstärken und der Signalverläufe der empfangenen Ultraschallsignale mittels der Auswerteeinrichtung ermittelt wird. Die Erfindung betrifft zudem ein medizinisches System zum Ausführen des Verfahrens.

## Beschreibung

Die Erfindung betrifft ein Verfahren und ein System zum Ermitteln einer Position einer Kanülenspitze im Inneren eines Körpers.

Solche Verfahren und Systeme finden unter anderem im Bereich der Regionalanästhesie Verwendung. Aus dem Stand der Technik ist ein System unter der Bezeichnung "OnVision" bekannt und dazu eingerichtet, die Position der Kanülenspitze in einer Bildebene eines Ultraschallbilds anzuzeigen. Das bekannte System ist vollständig in ein zugrunde liegendes bildgebendes Ultraschallsystem integriert.

Aufgabe der Erfindung ist es, ein Verfahren und ein System der eingangs genannten Art bereitzustellen, die Vorteile gegenüber dem Stand der Technik bieten.

Diese Aufgabe wird durch das Bereitstellen eines Verfahrens mit den Merkmalen des Anspruchs 1 und eines Systems mit den Merkmalen des Anspruchs 9 gelöst. Vorteilhafte Weiterbildungen sind in den Unteransprüchen angegeben. Der Wortlaut der Ansprüche wird durch Bezugnahme zum Gegenstand der Beschreibung gemacht.

Das erfindungsgemäße Verfahren ist zum Ermitteln einer Position einer Kanülenspitze im Inneren eines Körpers vorgesehen und weist folgende Schritte auf: Einkoppeln eines Ultraschallsignals in den Körper, wobei das Ultraschallsignal mit einem auf dem Körper aufliegenden Ultraschallkopf und entlang einer Bildebene eines bildgebenden Ultraschallverfahrens in den Körper eingekoppelt wird, wobei die Bildebene entlang einer Tiefenrichtung und einer Querrichtung erstreckt ist; Empfangen des Ultraschallsignals, wobei das Ultraschallsignal mit mehreren Sensoren empfangen wird, die an einer in dem Körper befindlichen Kanüle, welche die Kanülenspitze aufweist, angebracht und in bekannten axialen Abständen zu der Kanülenspitze und zueinander entlang einer Längsachse der Kanüle angeordnet sind; Vergleichen der empfangenen Ultraschallsignale, wobei die empfangenen Ultraschallsignale im Hinblick auf ihre Signalstärken und Signalverläufe mittels einer Auswerteeinrichtung verglichen werden, die mit den Sensoren verbunden ist; optionales Vergleichen der Signalstärken der empfangenen Ultraschallsignale mit einer an dem Ultraschallkopf anliegenden Signalstärke des Ultraschallsignals; Ermitteln der Position der Katheterspitze in Bezug auf den Ultraschallkopf, wobei die Position mittels der Auswerteeinrichtung und in Abhängigkeit des Vergleichs der Signalstärken und der Signalverläufe der empfangenen Ultraschallsignale und optional in Abhängigkeit des Vergleichs der empfangenen Signalstärken und der gesendeten Signalstärken ermittelt wird. Das erfindungsgemäße Verfahren hat den Vorteil, dass keine Integration in das bildgebende Ultraschallverfahren erforderlich ist. Das erfindungsgemäße Verfahren ist deshalb besonders universell einsetzbar. Die an dem Ultraschallkopf anliegende Signalstärke, d. h. die gesendete Signalstärke, kann beispielsweise als Nennwert in einer Speichereinheit der Auswerteeinrichtung hinterlegt sein. Es ist folglich keine Verbindung des Ultraschallkopfs mit der Auswerteeinrichtung erforderlich. Das erfindungsgemäße Verfahren erlaubt wenigstens ein näherungsweises Ermitteln der Position der Katheterspitze. Bei einer Ausgestaltung wird ein Axialabstand zwischen der Kanülenspitze und der Bildebene ermittelt. Bei einer weiteren Ausgestaltung wird alternativ oder zusätzlich ein Neigungswinkel der Kanüle in einer Vertikalebene ermittelt. Bei einer weiteren Ausgestaltung wird alternativ oder zusätzlich ein Tiefenabstand zwischen dem Ultraschallkopf und der Kanüle ermittelt. Bei einer weiteren Ausgestaltung wird alternativ oder zusätzlich ein Drehwinkel der Kanüle in einer Horizontalebene ermittelt. Anhand einzelner, mehrerer oder aller der vorgenannten Größen kann die Position der Katheterspitze in Bezug auf den Ultraschallkopf wenigstens näherungsweise ermittelt werden.

In Ausgestaltung der Erfindung umfasst das Ermitteln der Position ein Ermitteln eines Axialabstands zwischen der Kanülenspitze und der Bildebene. Der Axialabstand wird in Abhängigkeit des Vergleichs der Signalstärken der empfangenen Ultraschallsignale und der bekannten axialen Abstände der Sensoren mittels der Auswerteeinrichtung ermittelt. Durch den Vergleich der Signalstärken der empfangenen Ultraschallsignale kann ermittelt werden, welcher der mehreren Sensoren der Bildebene am nächsten liegt. Bei einer Positionierung unmittelbar in der Bildebene ist ein Maximum des empfangenen Ultraschallsignals zu erwarten. Folglich liegt derjenige Sensor, an welchem im Vergleich zu den weiteren Sensoren eine maximale Signalstärke des empfangenen Ultraschallsignals anliegt, am nächsten an der Bildebene. Da auch die axialen Abstände zwischen den Sensoren einerseits und andererseits den Sensoren der Kanülenspitze bekannt sind, kann auf Grundlage des Vergleichs ein Rückschluss auf den Axialabstand zwischen der Kanülenspitze und der Bildebene gezogen werden. Dabei ist der Axialabstand entlang der Längsachse der Kanüle erstreckt.

In weiterer Ausgestaltung der Erfindung umfasst das Vergleichen der Signalstärken ein Ermitteln desjenigen Sensors, an welchem im Vergleich zu den weiteren Sensoren eine maximale Signalstärke empfangen wird. Auch diese Ermittlung erfolgt mittels der Auswerteeinrichtung. Alternativ kann derjenige Sensor ermittelt werden, an welchem im Vergleich zu den weiteren Sensoren eine minimale Signalstärke empfangen wird. Der Sensor mit der minimalen Signalstärke wird in der Regel den größten Abstand zu der Bildebene entlang der Längsachse der Kanüle aufweisen.

In weiterer Ausgestaltung der Erfindung umfasst das Ermitteln der Position ein Ermitteln eines Neigungswinkels der Kanüle, wobei der Neigungswinkel auf eine Vertikalebene projiziert ist, die entlang der Tiefenrichtung und einer Längsrichtung erstreckt ist, und wobei der Neigungswinkel in Abhängigkeit des Vergleichs der Signalverläufe der empfangenen Ultraschallsignale und der bekannten axialen Abstände der Sensoren mittels der Auswerteeinrichtung ermittelt wird. Die Vertikalebene ist entlang der Tiefenrichtung der Bildebene und orthogonal zu der Bildebene erstreckt. Der Neigungswinkel wird in Abhängigkeit des Vergleichs der Signalverläufe der empfangenen Ultraschallsignale und der bekannten axialen Abstände der Sensoren ermittelt. Beispielsweise kann auf Grundlage des Vergleichs der Signalverläufe eine Phasenverschiebung und/oder eine Laufzeitdifferenz zwischen den empfangenen Signalen ermittelt werden. Diese Phasenverschiebung erlaubt einen Rückschluss auf einen Laufzeitunterschied des gesendeten Ultraschallsignals bis zu den einzelnen Sensoren. Bei gegebener Frequenz des Ultraschallsignals kann in Abhängigkeit der Phasenverschiebung zwischen den empfangenen Signalen von zwei oder mehr Sensoren auf deren Entfernung und/oder eine Entfernungsdifferenz zu dem Ultraschallkopf geschlossen werden. Alternativ oder zusätzlich wird die Laufzeitdifferenz direkt ausgewertet. Die Entfernungen oder Entfernungsdifferenzen und/oder die Laufzeitdifferenzen zusammen mit den bekannten axialen Abständen zwischen den Sensoren und der Kanülenspitze erlauben es, den Neigungswinkel zu ermitteln, beispielsweise auf Grundlage einfacher geometrischer, im Speziellen trigonometrischer, Beziehungen.

In weiterer Ausgestaltung der Erfindung umfasst das Ermitteln des Neigungswinkels ein Ermitteln eines Laufzeitunterschieds der empfangenen Ultraschallsignale für wenigstens einen ersten Sensor und einen zweiten Sensor der mehreren Sensoren, wobei der Laufzeitunterschied in Abhängigkeit des Vergleichs der Signalverläufe des ersten Sensors und des zweiten Sensors ermittelt wird. Bei dieser Ausgestaltung der Erfindung wird der Neigungswinkel in Abhängigkeit des Laufzeitunterschieds und des bekannten axialen Abstands zwischen dem ersten Sensor und dem zweiten Sensor ermittelt. Der Laufzeitunterschied wird in Abhängigkeit des Vergleiches der Signalverläufe der beiden Sensoren, oder auch noch mehrerer Sensoren, ermittelt. Der Laufzeitunterschied kann beispielsweise in Abhängigkeit einer Phasenverschiebung des mit dem ersten Sensor empfangenen Ultraschallsignals und des mit dem zweiten Sensor empfangenen Ultraschallsignals ermittelt werden. Alternativ oder zusätzlich kann die Laufzeit von dem Ultraschallkopf zu den Sensoren jeweils gemessen werden. Hierzu kann das bildgebende Ultraschallsystem mit der Auswerteeinrichtung verbunden sein. Alternativ dazu kann die Laufzeit zwischen dem Ultraschallkopf und dem Sensoren jeweils durch einen oder mehrere weitere Sensoren ermittelt werden, welcher unmittelbar an oder wenigstens nahe des Ultraschallkopfs angeordnet ist.

In weiterer Ausgestaltung der Erfindung umfasst das Verfahren einen Schritt Vergleichen der Signalstärken der empfangenen Ultraschallsignale mit einer an dem Ultraschallkopf anliegenden Signalstärke des Ultraschallsignals mittels der Auswerteeinrichtung, wobei das Ermitteln der Position weiter ein Ermitteln eines Tiefenabstands zwischen dem Ultraschallkopf und der Kanüle umfasst. Der Tiefenabstand ist in der Bildebene entlang der Tiefenrichtung erstreckt. Der Tiefenabstand wird in Abhängigkeit des Vergleichs der gesendeten Signalstärke des Ultraschallsignals und der empfangenen Signalstärken des Ultraschallsignals mittels der Auswerteeinrichtung ermittelt. Je größer der Unterschied zwischen der gesendeten Signalstärke des Ultraschallsignals und den Signalstärken der empfangenen Ultraschallsignale ist, desto geringer ist der Tiefenabstand zwischen der Kanüle und dem Ultraschallkopf. Umgekehrt gilt, dass der Tiefenabstand umso größer ist, je größer der Unterschied zwischen der gesendeten Signalstärke und den empfangenen Signalstärken ist. Das Ultraschallsignal wird innerhalb des Körpers gedämpft. Diese Dämpfung führt dazu, dass die Signalstärke mit zunehmendem Abstand von dem Ultraschallkopf in Tiefenrichtung abnimmt. Diesen Umstand macht sich diese Ausgestaltung der Erfindung zunutze. Die gesendete Signalstärke kann als Nennwert in einer Speichereinheit der Auswerteeinrichtung abgespeichert sein. Alternativ kann die gesendete Signalstärke gemessen werden. Weiter alternativ kann der Ultraschallkopf zum Übermitteln der gesendeten Signalstärke mit der Auswerteeinrichtung verbunden sein. In weiterer Ausgestaltung wird der Tiefenabstand in Abhängigkeit der Laufzeiten ermittelt. Hierzu muss der Sendezeitpunkt am Ultraschallkopf bekannt sein. Der Sendezeitpunkt kann beispielsweise durch einen Sensor in unmittelbarer Nähe des Ultraschallkopfs ermittelt werden. Alternativ oder zusätzlich kann der Ultraschallkopf mit der Auswerteeinrichtung verbunden sein, um die Sendezeit an die Auswerteeinrichtung zu übermitteln.

In weiterer Ausgestaltung der Erfindung umfasst das Ermitteln der Position ein Vergleichen eines axialen Verlaufs der empfangenen Signalstärken über der Länge der Kanüle mit Referenzdaten. Axial meint entlang der Längsachse der Kanüle. Die Referenzdaten repräsentieren wenigstens einen Verlauf der Signalstärke des Ultraschallsignals über der Längsrichtung und damit orthogonal zu der Tiefenrichtung und orthogonal zu der Querrichtung der Bildebene. Die Längsrichtung ist nicht zu verwechseln mit der Längsachse der Kanüle. Das Ermitteln der Position umfasst weiter ein Ermitteln eines Drehwinkels der Kanüle. Der Drehwinkel ist auf eine Horizontalebene projiziert, die entlang der Längsrichtung und der Querrichtung erstreckt ist. Die Horizontalebene ist somit orthogonal zu der Bildebene und orthogonal zu der bei der Ermittlung des Neigungswinkels relevanten Vertikalebene orientiert. Der Drehwinkel in der Horizontalebene wird in Abhängigkeit des Vergleichs zwischen dem axialen Verlauf der empfangenen Signalstärke und den Referenzdaten mittels der Auswerteeinrichtung ermittelt. Diese Ausgestaltung der Erfindung geht von der Überlegung aus, dass die Signalstärke des Ultraschallsignals mit zunehmendem orthogonalem Abstand von der Bildebene, d. h. entlang der Längsrichtung, abnimmt. Beträgt der Drehwinkel 0°, ist die Kanüle in der Bildebene längserstreckt. Davon ausgehend, dass der Neigungswinkel ebenfalls 0° beträgt, wird die Signalstärke der empfangenen Ultraschallsignale über der Länge der Kanüle (also entlang der Längsachse) in etwa identisch sein. Bei einer Drehung in der Horizontalebene verändert sich der Abstand der Sensoren zu der Bildebene entlang der Längsrichtung. Hierdurch ändert sich auch der Verlauf der Signalstärke des empfangenen Ultraschallsignals über der Längsachse der Kanüle. Durch den besagten Vergleich zwischen dem axialen Verlauf der empfangenen Signalstärke und den Referenzdaten, welche wenigstens den Verlauf der Signalstärke des Ultraschallsignals über der Längsrichtung repräsentieren, kann ein Rückschluss auf den Drehwinkel der Kanüle in der Horizontalebene gezogen werden. Die Referenzdaten sind vorzugsweise in einer Speichereinheit der Auswerteeinrichtung hinterlegt. Bei einer Ausgestaltung können die Referenzdaten aus einem Referenzdatensatz ausgewählt werden, der unterschiedliche Daten für unterschiedlich spezifizierte Ultraschallköpfe enthält. Die Auswahl kann durch einen Benutzer erfolgen. Bei einer weiteren Ausgestaltung werden die Referenzdaten durch eine Referenzmessung ermittelt. Alternativ oder zusätzlich können die Referenzdaten den Verlauf der Signalstärke in Abhängigkeit des Neigungswinkels und/oder des Drehwinkels repräsentieren.

In weiterer Ausgestaltung der Erfindung repräsentieren die Referenzdaten zudem einen Verlauf der Signalstärke des Ultraschallsignals entlang der Tiefenrichtung innerhalb der Bildebene, wobei der Drehwinkel zudem in Abhängigkeit des ermittelten Neigungswinkels und/oder des ermittelten Tiefenabstands ermittelt wird. Durch die Einbeziehung des Neigungswinkels und/oder des Tiefenabstands in die Ermittlung des Drehwinkels wird eine genauere Ermittlung des Drehwinkels und damit der Position der Kanülenspitze insgesamt ermöglicht.

Das erfindungsgemäße medizinische System ist zum Ermitteln einer Position einer Kanülenspitze in einem Körper eingerichtet. Das erfindungsgemäße medizinische System weist einen optionalen Ultraschallkopf, eine Kanüle und eine Auswerteeinrichtung auf. Der optionale Ultraschallkopf ist zum Einkoppeln eines Ultraschallsignals in den Körper eingerichtet. Die Kanüle weist die Kanülenspitze auf und ist mit mehreren Sensoren versehen. Die Sensoren sind jeweils zum Empfangen des Ultraschallsignals eingerichtet. Die Sensoren sind in bekannten axialen Abständen von der Kanülenspitze und zueinander entlang einer Längsachse der Kanüle angeordnet. Die Auswerteeinrichtung ist mit den Sensoren verbunden. Diese Verbindung kann drahtlos oder drahtgebunden sein. Die Auswerteeinrichtung ist zum Vergleichen der empfangenen Ultraschallsignale eingerichtet, insbesondere wenigstens im Hinblick auf die Signalstärken und/oder die Signalverläufe der empfangenen Ultraschallsignale. Optional ist die Auswerteeinrichtung zudem zum Vergleichen der Signalstärken der empfangenen Ultraschallsignale und einer an dem Ultraschallkopf anliegenden Signalstärke des Ultraschallsignals eingerichtet. Die Auswerteeinrichtung ist weiter zum Ermitteln der Position der Katheterspitze in Bezug auf den Ultraschallkopf in Abhängigkeit des Vergleichs der Signalstärken und der Signalverläufe der empfangenen Signale und optional in Abhängigkeit des Vergleichs der empfangenen Signalstärke und der gesendeten Signalstärke eingerichtet. Mit anderen Worten: Das System ist zum Ausführen des erfindungsgemäßen Verfahrens sowie dessen Ausgestaltungen eingerichtet. Bei einer Ausgestaltung weist das medizinische System den bereits erwähnten optionalen Ultraschallkopf auf. Bei einer weiteren Ausgestaltung weist das medizinische System keinen solchen Ultraschallkopf auf. In diesem Fall ist der Ultraschallkopf nicht Bestandteil des medizinischen Systems, sondern stattdessen Bestandteil eines bildgebenden Ultraschallsystems. Ausgestaltungen des medizinischen Systems ergeben sich aus den Merkmalen der Ausgestaltungen des erfindungsgemäßen Verfahrens, insbesondere im Hinblick auf eine etwaige weitergehende Einrichtung der Auswerteeinrichtung.

In weiterer Ausgestaltung der Erfindung ist die Auswerteeinrichtung weiter eingerichtet ist zum Vergleichen der Signalstärken der empfangenen Ultraschallsignale mit einer an dem Ultraschallkopf anliegenden Signalstärke des Ultraschallsignals, und zum Ermitteln der Position der Katheterspitze in Bezug auf den Ultraschallkopf in Abhängigkeit des Vergleichs der empfangenen Signalstärken und der gesendeten Signalstärke. Die an dem Ultraschallkopf anliegende Signalstärke kann auch gesendete Signalstärke bezeichnet werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Blockdarstellung eine Ausführungsform eines erfindungsgemäßen Verfahrens zum Ermitteln einer Position einer Kanülenspitze im Inneren eines Körpers,
- Fig. 2: in schematischer Blockdarstellung eine Ausführungsform eines erfindungsgemäßen medizinischen Systems, das zum Ausführen des Verfahrens nach Fig. 1 eingerichtet ist,
- Fig. 3: in schematisch vereinfachter Darstellung eine exemplarische Verwendungssituation des medizinischen Systems nach Fig. 2,
- Fig. 4: ein Diagramm mit exemplarischen Signalverläufen, die zur Ermittlung der Position der Kanülenspitze ausgewertet werden,
- Fig. 5: eine weitere exemplarische Verwendungssituation des medizinischen Systems nach Fig. 2,
- Fig. 6: ein weiteres Diagramm mit einem exemplarischen Signalverlauf,
- Fig. 7, 8, 9: weitere schematische Darstellungen exemplarischer Verwendungssituationen des medizinischen Systems nach Fig. 2,
- Fig. 10: ein Diagramm mit einem weiteren exemplarischen Signalverlauf,
- Fig. 11: in schematischer Darstellung eine weitere exemplarische Verwendungssituation des medizinischen Systems nach Fig. 2 und
- Fig. 12: ein Diagramm mit weiteren exemplarischen Signalverläufen.

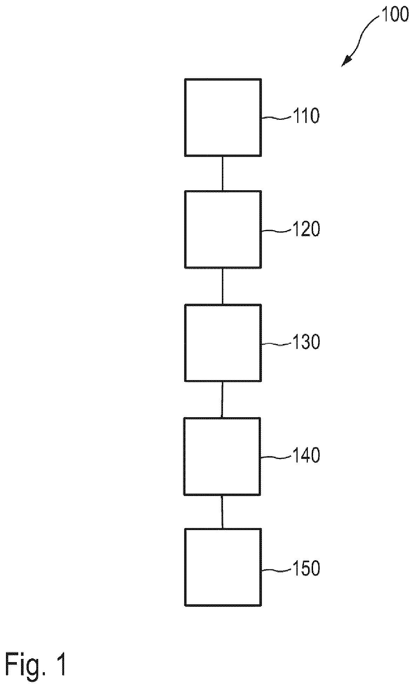

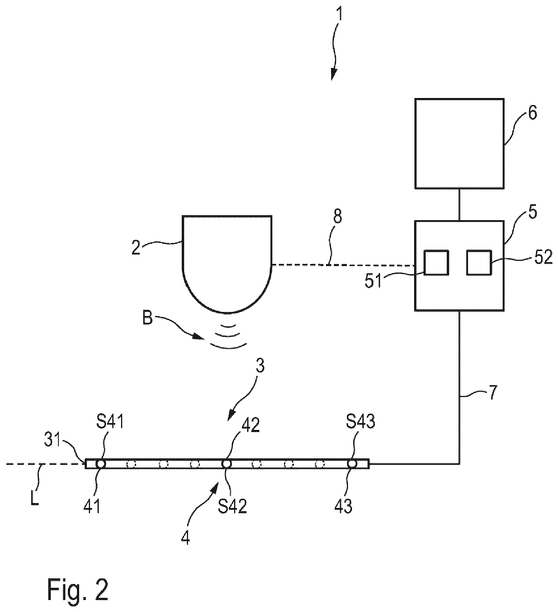

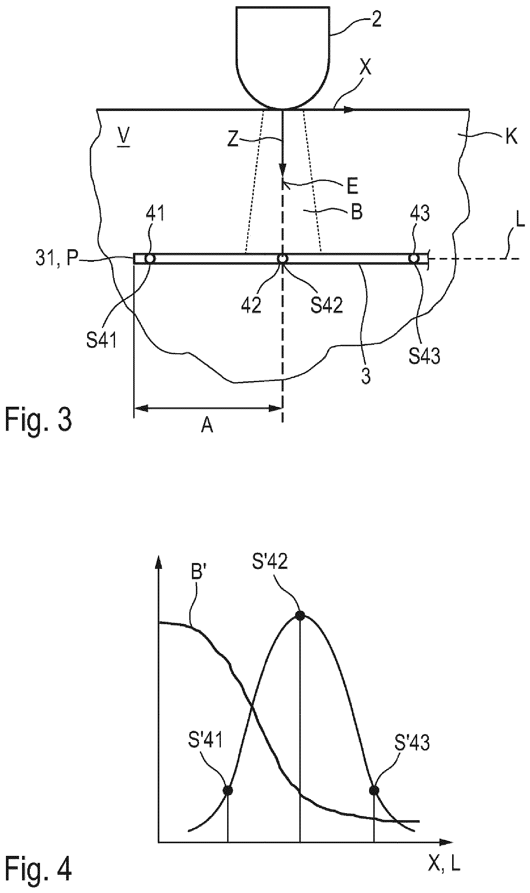

Gemäß Fig. 1 ist ein Verfahren 100 zum Ermitteln einer Position P einer Kanülenspitze 31 im Inneren eines Körpers K (siehe Fig. 3, 5, 7, 8, 9, 11) vorgesehen. Das Verfahren 100 kann mit dem in Fig. 2 gezeigten medizinischen System 1 ausgeführt werden, insbesondere im Rahmen einer Regionalanästhesie. Exemplarische Verwendungssituationen des medizinischen Systems 1 bei der Ausführung des Verfahrens 100 sind in den Fig. 3, 5 sowie 7 bis 9 und 11 gezeigt.

Das medizinische System 1 weist einen optionalen Ultraschallkopf 2, eine Kanüle 3, mehrere Sensoren 4, eine Auswerteeinrichtung 5 und eine optionale Anzeigeeinrichtung 6 auf. Der optionale Ultraschallkopf 2 und die optionale Anzeigeeinrichtung 6 sind nicht bei sämtlichen Ausführungsformen vorhanden.

Der Schritt 110 des Verfahrens 100 sieht ein Einkoppeln eines Ultraschallsignals B in den Körper K vor. Das Ultraschallsignal B wird mit dem Ultraschallkopf 2 abgegeben und in den Körper K eingekoppelt. In der Verwendung liegt der Ultraschallkopf 2 auf dem Körper K auf. Alternativ kann der Ultraschallkopf 2 auch in eine Öffnung des Körpers K eingeführt und im Inneren des Körpers K angeordnet sein. Das Ultraschallsignal B wird entlang einer Bildebene E in den Körper K eingekoppelt. Die Bildebene E ist entlang einer Tiefenrichtung Z und einer Querrichtung Y erstreckt.

Bei der in den Figuren gezeigten Ausführungsform ist der Ultraschallkopf 2 eine Komponente eines gesonderten bildgebenden Ultraschallsystems. Die besagte Bildebene E ist diejenige Ebene, in der ein Ultraschallbild des bildgebenden Ultraschallverfahrens erzeugt/angezeigt wird. Das Ultraschallsignal B kann in eingekoppeltem Zustand auch als Beam bezeichnet werden. Die Bezeichnung "Beam" ist der Fachperson bekannt.

Der Schritt 120 des Verfahrens 100 sieht ein Empfangen des Ultraschallsignals B vor. Das Ultraschallsignal B wird mit den mehreren Sensoren 4 empfangen. Die mehreren Sensoren 4 sind an der Kanüle 3 angebracht und in bekannten axialen Abständen zu der Kanülenspitze 31 sowie in bekannten Abständen zueinander entlang einer Längsachse L der Kanüle 3 angeordnet.

Die mehreren Sensoren 4 sind jeweils zum Empfangen des Ultraschallsignals B eingerichtet und können auch als Ultraschallmikrofone bezeichnet werden.

Bei der gezeigten Ausführungsform umfassen die mehreren Sensoren 4 einen ersten Sensor 41, einen zweiten Sensor 42 und einen dritten Sensor 43. Der erste Sensor 41 ist distal im Bereich der Kanülenspitze 31 angeordnet. Der zweite Sensor 42 ist entlang der Längsachse L zwischen dem ersten Sensor 41 und dem dritten Sensor 43 angeordnet und kann auch als medialer Sensor bezeichnet werden. Der dritte Sensor 43 ist proximal in Richtung eines proximalen Kanülenendes der Kanüle 3 angeordnet. Das proximale Kanülenende ist vorliegend nicht gezeigt.

Die vorliegende Anzahl von drei Sensoren ist rein exemplarisch. Es können auch mehr als drei Sensoren vorhanden sein, was durch die in Fig. 2 strichliert eingezeichneten weiteren Sensoren ohne Bezugszeichen verdeutlich wird.

In exemplarisch gezeigten Verwendungssituationen empfangen die Sensoren 41, 42, 43 das gesendete Ultraschallsignal B. Die an den Sensoren 41, 42, 43 anliegenden Ultraschallsignale S41, S42, S43 werden nachfolgend auch als erstes Ultraschallsignal S41, zweites Ultraschallsignal S42 und drittes Ultraschallsignal S43 bezeichnet. Das erste Ultraschallsignal S41 liegt an dem ersten Sensor 41 an. Das zweite Ultraschallsignal S42 liegt an dem zweiten Sensor 42 an. Das dritte Ultraschallsignal S43 liegt an dem dritten Sensor 43 an. Die Ultraschallsignale S41, S42, S43 unterscheiden sich hinsichtlich ihrer Signalstärke und/oder ihres Signalverlaufs. Diese Unterschiede sind abhängig von dem jeweiligen Abstand des betreffenden Sensors 41, 42, 43 von dem Ultraschallkopf 2 und damit von der Ultraschallquelle. Eine Auswertung der Signale S41, S42, S43 erlaubt damit eine Ermittlung der Position P, wobei auch die bekannten Abstände zwischen den Sensoren 41, 42, 43 und zu der Kanülenspitze 31 in die Ermittlung mit einbezogen werden.

Das Verfahren 100 sieht in dem Schritt 130 ein Vergleichen der empfangenen Ultraschallsignale S41, S42, S43 vor. Dieser Vergleich wird mittels der Auswerteeinrichtung 5 ausgeführt. Die Auswerteeinrichtung 5 ist zu diesem Zweck mittels einer Signalverbindung 7 mit den an der Kanüle 3 angebrachten Sensoren 4 verbunden. Die Signalverbindung 7 ist bei der gezeigten Ausführungsform eine Drahtverbindung. Bei einer in den Figuren nicht gezeigten Ausführungsform ist die Signalverbindung eine drahtlose Verbindung. Die mit den Sensoren 41, 42, 43 empfangenen Signale S41, S42, S43 werden mittels der Auswerteeinrichtung 5 im Hinblick auf ihre Signalstärken und Signalverläufe verglichen. Die Signalstärken der empfangenen Signale S41, S42, S43 werden nachfolgend mit einem Hochkomma gekennzeichnet und folglich als S'41, S'42, S'43 bezeichnet.

Das Verfahren 100 sieht in dem Schritt 140 ein Vergleichen der Signalstärken S'41, S'42, S'43 der empfangenen Ultraschallsignale S41, S42, S43 mit einer an dem Ultraschallkopf 2 anliegenden Signalstärke B' des Ultraschallsignals B vor. Die Signalstärke B' des gesendeten Ultraschallsignals B wird bei einer Ausführungsform gemessen. Bei einer weiteren Ausführungsform übermittelt der Ultraschallkopf 2 die Signalstärke B' an die Auswerteeinrichtung 5, beispielsweise über eine weitere Signalleitung 8. Diese weitere Signalleitung 8 ist optional und daher in Fig. 2 strichliert eingezeichnet. Bei einer weiteren Ausführungsform ist die Signalstärke B' des eingekoppelten Ultraschallsignals B als ein Nennwert des Ultraschallkopfs 2 gegeben. Dieser Nennwert kann beispielsweise in die Auswerteeinrichtung 5 eingegeben, von dieser empfangen oder in dieser gespeichert sein.

Das Verfahren 100 sieht in dem Schritt 150 ein Ermitteln der Position P der Katheterspitze 31 in Relation zu dem Ultraschallkopf 2 vor. Die Position P wird in Abhängigkeit des Vergleichs der Signalstärken S'41, S'42, S'43 und der Signalverläufe der empfangenen Ultraschallsignale S41, S42, S43 und in Abhängigkeit des Vergleichs der empfangenen Signalstärken S'41, S'42, S'43 mit der gesendeten Signalstärke B' des Ultraschallsignals B ermittelt. Die Ermittlung erfolgt mit der Auswerteeinrichtung 5.

Bei der gezeigten Ausführungsform weist die Auswerteeinrichtung 5 eine Prozessoreinheit 51 und eine Speichereinheit 52 auf. Die Prozessoreinheit 51 ist zum Ausführen der erwähnten Vergleiche 130, 140 und der eigentlichen Ermittlung 150 der Position P eingerichtet. In der Speichereinheit 52 ist bei der gezeigten Ausführungsform der Nennwert der Signalstärke B' des Ultraschallkopfs 2 hinterlegt. Dieser Nennwert bildet eine Art Referenzwert.

Die ermittelte Position P kann grafisch oder auf sonstige Weise mittels der Anzeigeeinrichtung 6 angezeigt werden. Die Anzeige kann beispielsweise in Bezug auf die bereits erwähnte Bildebene E erfolgen. Auch das eigentliche Ultraschallbild kann mittels der Anzeigeeinrichtung 6 angezeigt werden.

Die Anzeigeeinrichtung 6 kann alternativ oder zusätzlich zur akustisch wahrnehmbaren Anzeige oder aus Ausgabe der ermittelten Position P eingerichtet sein. Zu diesem Zweck kann die Anzeigeeinrichtung 6 eine Lautsprechereinheit aufweisen. Die ermittelte Position P oder deren Änderung kann in diesem Fall ähnlich wie bei einer Park-Distance-Control in einem PKW ausgegeben werden, beispielsweise mit einer Tonsignalfolge, Tonsignallautstärke, Tonsignalhöhe oder dergleichen.

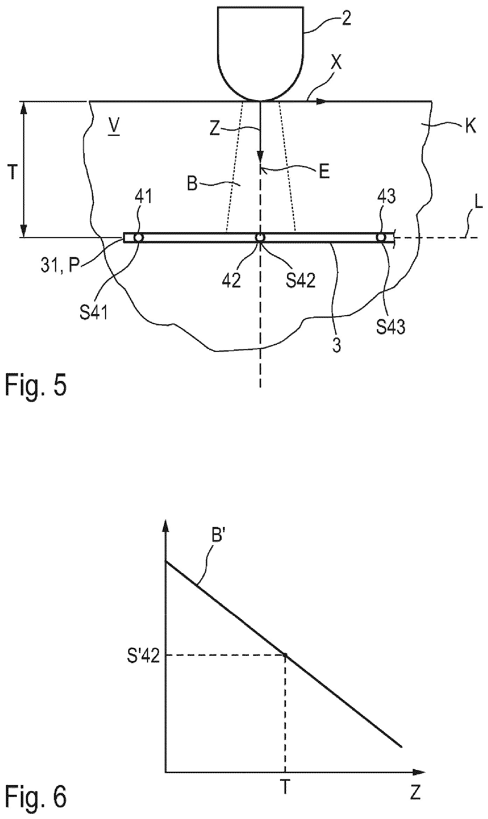

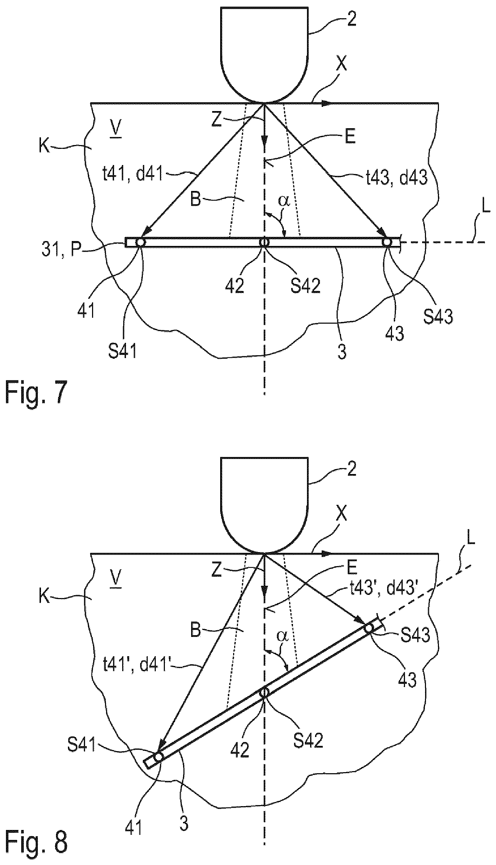

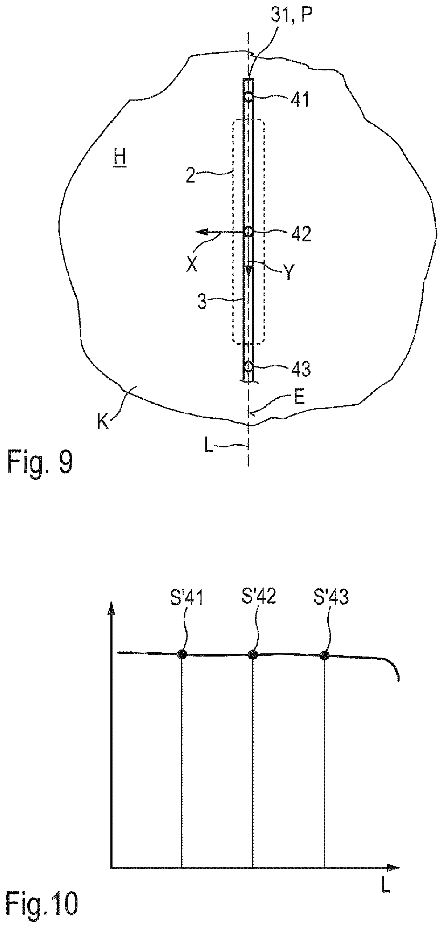

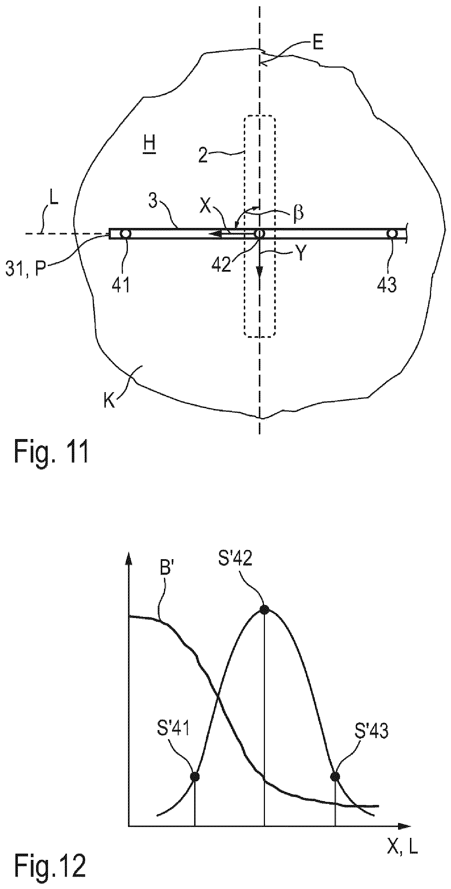

Das Verfahren 100 und das medizinische System 1 erlauben wenigstens eine näherungsweise Ermittlung der Position P. Näherungsweise meint, dass die Position P nicht hinsichtlich sämtlicher Koordinatenachsen eindeutig bestimmt sein muss. Beispielsweise kann mittels des Verfahrens 100 und des medizinischen Systems 1 lediglich ein Axialabstand A (siehe Fig. 3) zwischen der Kanülenspitze 31 und der Bildebene E ermittelt werden. Alternativ oder zusätzlich kann ein Tiefenabstand T ermittelt werden (siehe Fig. 5). Weiter alternativ oder zusätzlich kann ein Neigungswinkel α ermittelt werden (siehe Fig. 7, 8). Weiter alternativ oder zusätzlich kann ein Drehwinkel β ermittelt werden (siehe Fig. 9, 11). Bevorzugt werden sämtliche der vorgenannten Größen A, T, α, β ermittelt, so dass die Position P möglichst genau und/oder eindeutig bestimmt wird.

Der Axialabstand A zwischen der Kanülenspitze 31 und der Bildebene E wird in Abhängigkeit des Vergleichs der Signalstärken S'41, S'42, S'43 der empfangenen Ultraschallsignale S41, S42, S43 und der bekannten axialen Abstände der Sensoren 41, 42, 43 untereinander und in Bezug auf die Kanülenspitze 31 ermittelt. Die Ermittlung erfolgt mittels der Auswerteeinrichtung 5. Die Ermittlung des Axialabstands A geht von der Überlegung aus, dass der im geringsten Abstand zu der Bildebene E positionierte Sensor eine im Vergleich zu den weiteren Sensoren maximale Signalstärke liefert. In der in den Fig. 3 und 4 gezeigten exemplarischen Situation ist der zweite Sensor 42 unmittelbar in der Bildebene E positioniert. Die zweite Signalstärke S'42 ist folglich maximal im Vergleich zu den Signalstärken der weiteren Sensoren, insbesondere den Signalstärken S'41, S'43. Mit anderen Worten: Zur Ermittlung des Axialabstands A wird bestimmt, an welcher Längsposition der Kanüle 3 der Beam B positioniert ist. Der Axialabstand A ist entlang der Längsachse L der Kanüle 3 erstreckt. Bei der in Fig. 3 gezeigten exemplarischen Verwendungssituation ist die Längsachse L parallel zu einer Längsrichtung X erstreckt, die orthogonal zu der Tiefenrichtung Z und der Querrichtung Y der Bildebene E orientiert ist. In dem schematischen Diagramm nach Fig. 4 ist zusätzlich zu den Signalstärken S'41, S'42, S'43 der empfangenen Ultraschallsignale S41, S42, S43 ein exemplarischer Verlauf der Signalstärke B' des (gesendeten) Ultraschallsignals B über der Längsrichtung X eingezeichnet. Mit zunehmendem Abstand von der Bildebene E nimmt die gesendete Signalstärke B' ab. Dies aufgrund des Richtcharakters des Beams B und/oder der aus den Eigenschaften des Körpers K resultierenden Dämpfung.

Der Tiefenabstand T ist in der Bildebene E entlang der Tiefenrichtung Z erstreckt (siehe Fig. 5). Der Tiefenabstand T wird in Abhängigkeit des bereits erwähnten Vergleichs 140 der gesendeten Signalstärke B' und der empfangenen Signalstärke S'41, S'42, S'43 mittels der Auswerteeinrichtung 5 ermittelt. Die Ermittlung des Tiefenabstands T geht von der Überlegung aus, dass die Signalstärke B' des Beams B mit zunehmender Tiefe entlang der Tiefenrichtung Z abnimmt. Diese Abnahme ist vereinfacht und schematisch in dem exemplarischen Diagramm nach Fig. 6 gezeigt, wobei die exemplarische Darstellung logarithmisch ist. Der Verlauf der Signalstärke B' über der Tiefenrichtung Z ist bei der gezeigten Ausführungsform in Form von Referenzdaten in der Speichereinheit 52 der Auswerteeinrichtung 5 hinterlegt. Die Referenzdaten können beispielsweise mit einer Referenzmessung an einem Referenzmessaufbau vorab und/oder einmalig ermittelt werden.

Ist der Verlauf der Signalstärke B' des gesendeten Ultraschallsignals B über der Tiefenrichtung Z bekannt, kann über einen Vergleich mit den empfangenen Signalstärken S'41, S'42, S'43 auf den Tiefenabstand T geschlossen werden. Liegt beispielsweise der zweite Sensor 42, wie bei der exemplarischen Verwendungssituation, unmittelbar in der Bildebene E, kann der Tiefenabstand T zwischen dem zweiten Sensor 42 und dem Ultraschallkopf 2 entlang der Tiefenrichtung Z über einen einfachen Vergleich zwischen der zweiten Signalstärke S'42 und dem besagten Verlauf der Signalstärke B' über der Tiefenrichtung Z bestimmt werden.

Sofern die Kanüle 3 geneigt angeordnet sein sollte (siehe beispielsweise Fig. 8), kann der Tiefenabstand der Kanülenspitze 31 über einfache trigonometrische Beziehungen und auf Grundlage der bekannten Abstände zwischen den Sensoren 41, 42, 43 und der Kanülenspitze 31 ermittelt werden.

Der Neigungswinkel α der Kanüle 3 ist auf eine Vertikalebene V projiziert, die entlang der Tiefenrichtung Z und der Längsrichtung X erstreckt ist (siehe Fig. 7, 8). Der Neigungswinkel α wird in Abhängigkeit des Vergleichs der bereits erwähnten Signalverläufe der empfangenen Ultraschallsignale S41, S42, S43 und der bekannten axialen Abstände der Sensoren 41, 42, 43 untereinander und in Bezug auf die Kanülenspitze 31 ermittelt. Auch die Ermittlung des Neigungswinkels α erfolgt mittels der Auswerteeinrichtung 5.

Die Ermittlung des Neigungswinkels α geht von der Überlegung aus, dass je nach Abstand der Sensoren 41, 42, 43 von dem Ultraschallkopf 2 sich unterschiedliche Laufzeiten des Ultraschallsignals B ergeben. Diese unterschiedlichen Laufzeiten sind schematisch in den Fig. 7 und 8 für den ersten Sensor 41 und den dritten Sensor 43 jeweils in Pfeilform eingezeichnet. Die Laufzeit des Ultraschallsignals B zwischen dem Ultraschallkopf 2 und dem ersten Sensor 41 wird als t41 bezeichnet. Die dem dritten Sensor 43 zugeordnete Laufzeit wird mit t43 bezeichnet. In der exemplarischen Verwendungssituation nach Fig. 7 ist die Längsachse L parallel zu der Längsrichtung X orientiert und die beiden Sensoren 41, 43 sind in identischen axialen Abständen entlang der Längsachse L zu der Bildebene E positioniert. Die Laufzeiten t41, t43 sind folglich identisch. Auch die Abstände zwischen dem Ultraschallkopf 2 und den beiden Sensoren 41, 43 sind identisch. Die besagten Abstände sind in Fig. 7 mit den Bezugszeichen d41, d43 belegt und ergeben sich rein rechnerisch aus der Laufzeit des gesendeten Ultraschallsignals B und dessen Frequenz. Die Frequenz kann beispielsweise mittels der Auswerteeinrichtung 5 anhand der empfangenen Signale S41, S42, S43 ermittelt werden. Alternativ oder zusätzlich kann die Frequenz des Ultraschallsignals B als Nennwert in der Speichereinheit 52 der Auswerteeinrichtung 5 hinterlegt sein. Bei der in Fig. 7 gezeigten exemplarischen Verwendungssituation existiert folglich keine Phasenverschiebung zwischen den Signalverläufen der empfangenen Ultraschallsignale S41, S43.

Anders liegt es in der in Fig. 8 gezeigten Verwendungssituation. Dort ist die Kanüle 3 ausgehend von der horizontalen Ausrichtung nach Fig. 7 distal in Tiefenrichtung T nach unten geneigt. Der dritte Sensor 43 liegt folglich näher an dem Ultraschallkopf 2 als der erste Sensor 41. Folglich ergeben sich unterschiedliche Laufzeiten t41', t43' und damit auch unterschiedliche Abstände d41', d43'. Dabei werden die Laufzeiten t41, t43 bzw. t41', t43' nicht über eine unmittelbare Zeitmessung bestimmt. Vielmehr erfolgt eine mittelbare Bestimmung über den Vergleich der Signalverläufe der Signale S41, S43 und eine sich ergebende Phasenverschiebung zwischen diesen beiden Signalen S41, S43. Der Neigungswinkel α kann auf Grundlage des Unterschieds zwischen den Laufzeiten und einfacher trigonometrischer Beziehungen ermittelt werden.

Ist der Neigungswinkel α bekannt, können der axiale Abstand A und/oder der Tiefenabstand T mit verbesserter Genauigkeit ermittelt werden und umgekehrt. Dies wiederum auf der Grundlage einfacher trigonometrischer Beziehungen und in Abhängigkeit der bekannten Abstände zwischen den Sensoren 41, 42, 43 und deren Abständen zu der Kanülenspitze 31.

Der Drehwinkel β (siehe Fig. 9) ist ein auf einer Horizontalebene H projizierter Winkel. Die Horizontalebene H ist orthogonal zu der Bildebene E und der Vertikalebene V orientiert. Die Horizontalebene H ist folglich entlang der Längsrichtung X und der Querrichtung Y erstreckt.

Der Ultraschallkopf 2 ist in den Fig. 9 und 11 strichliert eingezeichnet und entlang der Querrichtung Y längserstreckt. Der Ultraschallkopf 2 ist länglich in Bezug auf die Querrichtung Y. Mit anderen Worten: Die Abmessungen entlang der Querrichtung Y sind deutlich größer als entlang der Längsrichtung X.

Die Ermittlung des Drehwinkels β geht wiederum von der Überlegung aus, dass die Signalstärke B' des eingekoppelten Ultraschallsignals B, d. h. des Beams, mit zunehmendem Abstand von der Bildebene E und damit entlang der Längsrichtung X abnimmt. Diese exemplarische Abnahme der Signalstärke B' ist in Fig. 12 schematisch gezeigt.

In der exemplarischen Verwendungssituation nach Fig. 9 ist der Drehwinkel β in etwa null und folglich ist die Längsachse L parallel zu der Querrichtung Y orientiert. Eine solche Längserstreckung der Kanüle 3 innerhalb der Bildebene E wird auch als eine Anordnung "in plane" bezeichnet. Davon ausgehend, dass die Signalstärke B' des Beams B in Längsrichtung Y - jedenfalls über der Länge des Ultraschallkopfs 2 - unveränderlich ist, ergeben sich bei einer Anordnung "in plane" auch in etwa identische Signalstärken S'41, S'42, S'43 entlang der Längsachse L (siehe Fig. 10).

Mit zunehmendem Drehwinkel β nimmt auch der Unterschied zwischen den Signalstärken S'41, S'42, S'43 zu. Dieser Unterschied wird bei einem Drehwinkel β von 90° maximal (siehe Fig. 11, 12, Anordnung "out of plane"). Der Drehwinkel β kann folglich über ein Vergleichen eines axialen Verlaufs der empfangenen Signalstärken S'41, S'42, S'43 über der Länge der Kanüle 3, d. h. entlang der Längsachse L, mit dem Verlauf der Signalstärke B über der Längsrichtung X ermittelt werden. Der Verlauf der Signalstärke B' des Ultraschallsignals B über der Längsrichtung X ist vorliegend in Form von Referenzdaten in der Speichereinheit 52 abgespeichert.

## Patentansprüche

1. Verfahren (100) zum Ermitteln einer Position (P) einer Kanülenspitze (31) im Inneren eines Körpers (K), aufweisend die Schritte:
Einkoppeln (110) eines Ultraschallsignals (B) in den Körper (K), wobei das Ultraschallsignal (B) mit einem auf den Körper (B) aufliegenden Ultraschallkopf (2) und entlang einer Bildebene (E) eines bildgebenden Ultraschallverfahrens in den Körper (K) eingekoppelt wird, wobei die Bildebene (E) entlang einer Tiefenrichtung (Z) und einer Querrichtung (Y) erstreckt ist;
Empfangen (120) des Ultraschallsignals (B), wobei das Ultraschallsignal (B) mit mehreren Sensoren (41, 42, 43) empfangen wird, die an einer in dem Körper befindlichen Kanüle (3) angebracht und in bekannten axialen Abständen zu der Kanülenspitze (31) und zueinander entlang einer Längsachse (L) der Kanüle (3) angeordnet sind;
Vergleichen (130) der empfangenen Ultraschallsignale (S41, S42, S43), wobei die empfangenen Ultraschallsignale (S41, S42, S43) im Hinblick auf ihre Signalstärken (S'41, S'42, S'43) und ihre Signalverläufe mittels einer Auswerteeinrichtung (5) verglichen werden, die mit den Sensoren (41, 42, 43) verbunden ist; und
Ermitteln (150) der Position (P) der Katheterspitze (31) in Bezug auf den Ultraschallkopf (2), wobei die Position (P) in Abhängigkeit des Vergleichs der Signalstärken (S'41, S'42, S'43) und der Signalverläufe der empfangenen Ultraschallsignale (S41, S42, S43) mittels der Auswerteeinrichtung (5) ermittelt wird.

2. Verfahren (100) nach Anspruch 1, wobei das Ermitteln (150) der Position (P) weiter umfasst:
Ermitteln eines Axialabstands (A) zwischen der Kanülenspitze (31) und der Bildebene (E), wobei der Axialabstand (A) in Abhängigkeit des Vergleichs der Signalstärken (S'41, S'42, S'43) der empfangenen Ultraschallsignale (S41, S42, S43) und der bekannten axialen Abstände der Sensoren (41, 42, 43) mittels der Auswerteeinrichtung (5) ermittelt wird.

3. Verfahren nach Anspruch 2, wobei das Vergleichen der Signalstärken (S'41, S'42, S'43) weiter umfasst:
Ermitteln desjenigen Sensors (42), an welchem im Vergleich zu den weiteren Sensoren (41, 43) eine maximale Signalstärke (S'42) empfangen wird.

4. Verfahren (100) nach einem der vorhergehenden Ansprüche, wobei das Ermitteln (150) der Position (P) weiter umfasst:
Ermitteln eines Neigungswinkels (α) der Kanüle (3), wobei der Neigungswinkel (α) auf eine Vertikalebene (V) projiziert ist, die entlang der Tiefenrichtung (Z) und einer Längsrichtung (X) erstreckt ist, und wobei der Neigungswinkel (α) in Abhängigkeit des Vergleichs der Signalverläufe der empfangenen Ultraschallsignale (S41, S42, S43) und der bekannten axialen Abstände der Sensoren (41, 42, 43) mittels der Auswerteeinrichtung (5) ermittelt wird.

5. Verfahren (100) nach Anspruch 4, wobei das Ermitteln des Neigungswinkels (α) weiter umfasst:
Ermitteln eines Laufzeitunterschieds der empfangenen Ultraschallsignale (S41, S42, S43) für wenigstens einen ersten Sensor (41) und einen zweiten Sensor (43) der mehreren Sensoren (41, 42, 43), wobei der Laufzeitunterschied in Abhängigkeit des Vergleichs der Signalverläufe des ersten Sensors (41) und des zweiten Sensors (43) ermittelt wird, und
Ermitteln des Neigungswinkels (α) in Abhängigkeit des Laufzeitunterschieds und des bekannten axialen Abstands zwischen dem ersten Sensor (41) und dem zweiten Sensor (43).

6. Verfahren (100) nach einem der vorhergehenden Ansprüche, weiter aufweisend einen Schritt
Vergleichen (140) der Signalstärken (S'41, S'42, S'43) der empfangenen Ultraschallsignale (S41, S42, S43) mit einer an dem Ultraschallkopf (2) anliegenden Signalstärke (B') des Ultraschallsignals (B), wobei das Vergleichen (140) mit der Auswerteeinrichtung (5) erfolgt;
wobei das Ermitteln (150) der Position (P) weiter umfasst:
Ermitteln eines Tiefenabstands (T) zwischen dem Ultraschallkopf (2) und der Kanüle (3), wobei der Tiefenabstand (T) in der Bildebene (E) entlang der Tiefenrichtung (Z) erstreckt ist, und wobei der Tiefenabstand (T) in Abhängigkeit des Vergleichs der an dem Ultraschallkopf (2) anliegenden Signalstärke (B') und der empfangenen Signalstärken (S'41, S'42, S'43) mittels der Auswerteeinrichtung (5) ermittelt wird.

7. Verfahren (100) nach einem der vorhergehenden Ansprüche, wobei das Ermitteln (150) der Position (P) weiter umfasst:
Vergleichen eines axialen Verlaufs der empfangenen Signalstärken (S'41, S'42, S'43) über der Länge der Kanüle (3) mit Referenzdaten, die wenigstens einen Verlauf der Signalstärke (B') des Ultraschallsignals (B) über der Längsrichtung (X) repräsentieren;
Ermitteln eines Drehwinkels (β) der Kanüle (3), wobei der Drehwinkel (β) auf eine Horizontalebene (H) projiziert ist, die entlang der Längsrichtung (X) und der Querrichtung (Y) erstreckt ist, und wobei der Drehwinkel (β) in Abhängigkeit des Vergleichs zwischen dem axialen Verlauf der empfangenen Signalstärken (S'41, S'42, S'43) und den Referenzdaten mittels der Auswerteeinrichtung (5) ermittelt wird.

8. Verfahren (100) nach Anspruch 7 in Kombination mit Anspruch 4 und/oder 6, wobei die Referenzdaten zudem einen Verlauf der Signalstärke (B') des Ultraschallsignals (B) entlang der Tiefenrichtung (Z) innerhalb der Bildebene (E) repräsentieren, und wobei der Drehwinkel (β) zudem in Abhängigkeit des ermittelten Neigungswinkels (α) und/oder des ermittelten Tiefenabstands (T) ermittelt wird und/oder umgekehrt.

9. Medizinisches System (1) zum Ermitteln einer Position (P) einer Kanülenspitze (31) in einem Körper (K), aufweisend
einen Ultraschallkopf (2), der zum Einkoppeln eines Ultraschallsignals (B) in den Körper (K) eingerichtet ist,
eine Kanüle (3) mit der Kanülenspitze (31) und mit mehreren Sensoren (41, 42, 43), die zum Empfangen des Ultraschallsignals (B) eingerichtet sind, und die in bekannten axialen Abständen zu der Kanülenspitze (31) und zueinander entlang einer Längsachse (L) der Kanüle (3) angeordnet sind,
eine Auswerteeinrichtung (5), die mit den Sensoren (41, 42, 43) verbunden ist, wobei die Auswerteeinrichtung (5) eingerichtet ist zum
Vergleichen (130) der empfangenen Ultraschallsignale (S41, S42, S43), insbesondere wenigstens im Hinblick auf ihre Signalstärken (S'41, S'42, S'43) und ihre Signalverläufe, und
Ermitteln (150) der Position (P) der Katheterspitze (31) in Bezug auf den Ultraschallkopf (2) in Abhängigkeit des Vergleichs (130) der Signalstärken (S'41, S'42, S'43) und der Signalverläufe der empfangenen Signale (S41, S42, S43).

10. Medizinisches System (1) nach Anspruch 9, wobei die Auswerteeinrichtung (5) weiter eingerichtet ist zum
Vergleichen (140) der Signalstärken (S'41, S'42, S'43) der empfangenen Ultraschallsignale (S41, S42, S43) mit einer an dem Ultraschallkopf (2) anliegenden Signalstärke (B') des Ultraschallsignals (B), und
Ermitteln (150) der Position (P) der Katheterspitze (31) in Bezug auf den Ultraschallkopf (2) in Abhängigkeit des Vergleichs (140) der empfangenen Signalstärken (S'41, S'42, S'43) und der gesendeten Signalstärke (B').
